(19) Europäisches Patentamt
European Patent Office
Office européen des brevets

(11) Publication number: **0 017 246**
**B1**

(12) **EUROPEAN PATENT SPECIFICATION**

(45) Date of publication of patent specification: **11.04.84**

(21) Application number: **80101825.0**

(22) Date of filing: **03.04.80**

(51) Int. Cl.³: **C 07 D 487/04,**
C 12 P 17/18, A 61 K 31/40
//(C07D487/04, 209/00,
205/00)

# (54) Antibiotic PA-31088-IV, its salts with bases, a process for its manufacture, pharmaceutical compositions containing this antibiotic, streptomyces tokunonensis PA-31088 and its mutants.

(30) Priority: **06.04.79 JP 42347/79**

(43) Date of publication of application:
**15.10.80 Bulletin 80/21**

(45) Publication of the grant of the patent:
**11.04.84 Bulletin 84/15**

(84) Designated Contracting States:
**CH DE FR GB IT LI NL SE**

(56) References cited:
**EP - A - 0 005 349**
**DE - A - 2 815 157**

(73) Proprietor: **SHIONOGI & CO., LTD.**
**12, Dosho-machi 3-chome Higashi-ku**
**Osaka 541 (JP)**

(72) Inventor: **Tanaka, Kentaro**
**3-19-2, Furuedai**
**Suita-shi Osaka Pref. (JP)**
Inventor: **Shoji, Jun'ichi**
**1-17-14, Nagaodai**
**Hirakata-shi Osaka Pref. (JP)**
Inventor: **Kawamura, Yoshimi**
**1971-9, Aomadani**
**Mino-shi Osaka Pref. (JP)**
Inventor: **Hattori, Teruo**
**4-9-5, Nakasujiyamate**
**Takarazuka-shi Hyogo Pref. (JP)**
Inventor: **Kondo, Eiji**
**4-22-18, Ishibashi**
**Ikeda-shi Osaka Pref. (JP)**
Inventor: **Matsumoto, Kouichi**
**6-11-72-401, Higashitoyonaka-cho**
**Toyonaka-shi Osaka Pref. (JP)**
Inventor: **Yoshida, Tadashi**
**6-5-19-201, nogami**
**Takarazuka-shi Hyogo Pref. (JP)**
Inventor: **Tsuji, Naoki**
**1-326, Okuyama**
**Ashiya-shi Hyogo Pref. (JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 017 246 B1**

Courier Press, Leamington Spa, England.

**0 017 246**

(74) Representative: **Vossius Vossius Tauchner Heunemann Rauh**
**Siebertstrasse 4 P.O. Box 86 07 67**
**D-8000 München 86 (DE)**

Antibiotic PA—31088—IV, its salts with bases, a process for its manufacture, pharmaceutical compositions containing this antibiotic, Streptomyces tokunonensis PA—31088 and its mutants

This invention relates to a new antibiotic PA—31088—IV and its salts with bases, and a process for preparing the antibiotic by cultivating a strain of Streptomyces tokunonensis sp. nov. in a suitable medium and recovering PA—31088—IV from the cultured broth.

This invention also relates to the use of the antibiotic for use in the antibiotic treatment and to pharmaceutical compositions. This invention furthermore relates to a novel microorganism belonging to the genus Streptomyces and its mutants which produces in a culture medium said antibiotic.

The antibiotic PA—31088—IV inhibits the growth of both gram-positive and gram-negative pathogenic microorganisms. Furthermore, it shows a wide range of β-lactamase inhibitory activity.

From DE—A—2 815 157 3-(2-aminoethylthio)-6-ethyl-7-oxo-1-axabicyclo-[3.2.0]-hept-2-ene-2-carboxylic acid is known which exhibits antibiotic activity against certain gram-positive and gram-negative bacteria. It is not disclosed whether this compound shows β-lactamase inhibition.

Several antibiotics are known to have a β-lactamase inhibitory activity. They include clavuranic acid (disclosed in Examined Japanese Patent Publication (Jap. Pat. Pub.) No. 52-1996), MC 696—$SY_2$ (Unexamined Jap. Pat. Pub. No. 54-14594), MM—4550 (Unexamined Jap. Pat. Pub. No. 52-83994), MM—13902 (Unexamined Jap. Pat. Pub. No. 52-83992), MM—17880 (Unexamined Jap. Pat. Pub. No. 51-118701), and PS—5 (Unexamined Jap. Pat. Pub. No. 53—121702). Additionally, it is supposed that thienamycin, N-acetylthienamycin, epithienamycins No. $17927A_1$ (disclosed in Unexamined Jap. Pat. Pub. No. 53-103401), and $17927A_2$ (unexamined Jap. Pat. Pub. No. 53-109997) have almost the same activity. The chemical structures of some of these antibiotics are known and disclosed in J. Antibiotics, Vol. 32, No. 9 (1979), pp. 961—963.

The antibiotic PA—31088—IV has the following structural formula:

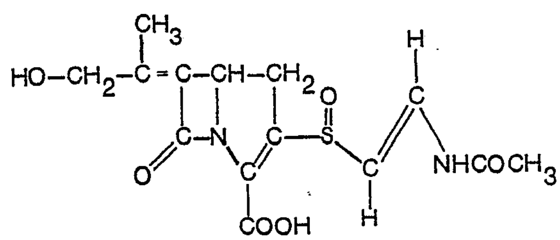

It is, therefore a 3 - [(2 - acetamidoethenyl) - sulfinyl] - 6 - (2-hydroxy - 1 - methylethylidene) - 7 - oxo - 1 - azabicyclo[3.2.0]hept - 2 - ene - 2 - carboxylic acid.

The above structure is different from those of known antibiotics having β-lactamase inhibitory activity. Additionally, the physicochemical properties of PA—31088—IV described below prove that PA—31088—IV is different from any other known β-lactam antibiotic.

The antibiotic PA—31088—IV has the following physicochemical and biological properties:

(1) Physicochemical properties of PA—31088—IV

(a) Elemental analysis
Anal. calcd. for $C_{14}H_{15}N_2O_6S.Na$

| | | | | | |
|---|---|---|---|---|---|
| | C, 46.41; | H, 4.14; | N, 7.73; | S, 8.84; | Na, 6.35 |
| Found: | C, 45.30; | H, 4.82; | N, 7.74; | S, 7.86; | Na, 5.99 |

(b) Mass spectrum of the methyl ester: m/e: 355

(c) Circular dichroism spectrum:
$\lambda_{(nm)}[\theta]$ 390(0), 315(−22800), 278(−103000), 258.5(0), 243(+93300), 190(+14500)

(d) Ultraviolet absorption spectrum (in 10 mM phosphate buffer):
$\lambda_{max}^{H_2O}$ 241 nm ($E_{1\,cm}^{1\%}$ 562) (see Fig. 1)

(e) Infrared absorption spectrum:
$\nu_{KBr}$ 3380, 1750, 1695, 1620, 1380, 1270, 1200, 1045, 970 $cm^{-1}$ (see Fig. 2)

(f) Solubility:
Soluble in water, not soluble in acetone, ethyl acetate, chloroform and ether.

(g) Color reaction:
Ninhydrin reaction: negative
Ehrlich's reaction: positive

(h) Acidic substance

(i) $^1H$—NMR spectrum (TMS external reference):
$\delta^{D_2O}_{ppm}$ (Hz) 2.44 (s, 3H), 2.57 (s, 3H), 3.62 (d-like, splitting width 9.4 Hz, 2H), 4.71 (s, 2H), 5.45 (t-like, splitting width 9.4 Hz and 9.4 Hz, 1H), 6.79 (d, 1H, J=14.1), 7.98 (d, 1H, J=14.1)

(j) $^{13}C$—NMR spectrum:
$\delta^{D_2O}_{ppm}$: 15.9q, 23.0q, 32.5t, 60.4t, 64.5t, 111.7d, 134.2s, 134.7d, 135.6s, 143.2s, 150.3s, 166.7s, 173.2s, 174.6s

(k) Thin layer chromatography:
Rf=0.73 (70% ethanol)
Rf=0.30 (80% acetonitrile)
Rf=0.50 (chloroform/ethanol/water (4:7:2))
on cellulose plate (Eastman Co., Ltd.).

(2) Biological properties of PA—31088—IV

(a) Antibacterial spectrum

| Test Bacteria | Minimum Inhibitory Concentration (μg/ml) |
|---|---|
| Staphylococcus aureus 209P JC–1 | 3.13 |
| Streptococcus pneumoniae I | 0.39 |
| Escherichia coli NIHJ JC–2 | 3.13 |
| Klebsiella pneumoniae SRL–1 | 1.56 |
| Klebsiella sp. 363 (R) | 3.13 |
| Proteus mirabilis PR–4 | 6.25 |
| Enterobacter cloacae 233 | 3.13 |
| Serratia marcescens ATCC 13880 | 12.5 |
| Pseudomonas aeruginosa ATCC 25619 | 50 |

Note: Inoculum size = $10^6$ colony forming units/ml.

(b) β-Lactamase inhibitory activity

| β-Lactamase Producing Bacteria | Minimum Inhibitory Concentration (μg/ml) |
|---|---|
| Enterobacter cloacae 92* | 0.98 |
| Klebsiella sp. 363** | 0.06 |

Notes: * produces β-lactamase of cephalosporinase type

** produces β-lactamase of penicillinase type

(c) Synergistic activity with other β-lactam antibiotics

| PA—31088—IV (μg/ml) | MIC of Ampicillin (μg/ml) | | |
|---|---|---|---|
| | S. aureus 2132 | P. vulgaris 50 | S. marcescens 39 |
| 0 | 100 | > 200 | > 200 |
| 2.5 | 12.5 | 6.3 | 3.1 |
| 10 | 6.3 | 1.6 | 0.1 |
| | MIC of Cefazolin (μg/ml) | | |
| 0 | 6.3 | > 200 | > 200 |
| 2.5 | 0.8 | 6.3 | 6.3 |
| 10 | 0.4 | 3.1 | 0.4 |
| MIC of PA—31088—IV | > 10 | > 10 | > 10 |

The antibiotic PA—31088—IV is produced by a microorganism belonging to the genus *Streptomyces*. The microorganism was isolated from a soil sample collected at Tokunoshima in Kagoshima Pref., Japan, and tentatively named strain PA—31088. After the taxonomical studies described below, the strain was designated *Streptomyces tokunonensis* PA—31088 and deposited with the Fermentation Research Institute of Agency of Industrial Science & Technology, Yatabe-machi, Ibaragi, Japan, 300—21, under the accession number, FERM—P No. 4843 and with American Type Culture Collection, 12301 Parklawn Drive, Rockville, Maryland 20952, U.S.A. under the accession number, ATCC No. 31569.

The strain *Streptomyces tokunonensis* PA—31088 has the following characteristics:

(a) Morphological properties (cultured on Bennett's agar at 28°C for 14 days)

No sporangium, flagellated spore or sclerotium are observed. Basal hyphae are not split by fragmentation. The aerial hyphae ar formed in abundance on this medium. Conidia are produced on the aerial hyphae and simply branched from the main axis to form branches, the ends of which are spiral. The surface of the spore is smooth and the spore is seen under electron microscopy to be a short cylinder.

(b) Cultural properties (cultured at 28°C for 14 days)

| Medium | Growth | Aerial Hyphae | | Color of Basal Hyphae | Soluble Pigment |
|---|---|---|---|---|---|
| | | Form | Color | | |
| Sucrose Nitrate Agar Medium | Fair | Fair | Pale brown | Pale yellowish brown | None |
| Glucose Asparagine Agar Medium | ,, | None | – | ,, | ,, |
| Glycerine Asparagine Agar Medium | ,, | Little | White | ,, | ,, |
| Inorganic salt Starch Agar Medium | Good | Good | Pale reddish orange | ,, | ,, |
| Tyrosine Agar Medium | ,, | Fair | Pale brown | ,, | ,, |
| Nutrient Agar Medium | Fair | None | – | ,, | ,, |
| Yeast extract Malt extract Agar Medium | Good | Good | Pale reddish orange | ,, | ,, |
| Oatmeal Agar Medium | ,, | ,, | ,, | ,, | ,, |
| Bennett's Agar Medium | ,, | ,, | ,, | ,, | ,, |

Note: The expression of the color is based on "The Standard of the Color" (Japan Color Institute).

(c) Physiological properties
Liquefaction of gelatin: poor growth
Hydrolysis of starch: positive
Tyrosinase activity: negative
Melanoid chromogenic function: negative
Peptonization of milk: positive
Coagulation of milk: negative
Utilization of carbohydrates
Carbohydrates producing good growth:
glucose, inositol
Carbohydrates producing no growth:
arabinose, xylose, fructose, sucrose, rhamnose, raffinose, mannitol

Growth temperature
- 10°C no growth
- 37°C fair growth but no aerial hyphae and spores are formed
- 42°C no growth
- 45°C no growth
- 50°C no growth

From the above properties it is clear that the strain PA—31088 belongs to the genus *Streptomyces*.

*Streptomyces fradiae* and *Streptomyces tenebrarius* are similar to the strain PA—31088 in that the aerial hyphae are Red color series, the spore chain is spiral, the surface of the spore is smooth, and melanoid chromogenic function is negative, judging from the description in "The Actinomycetes" *2* (1961) by Waksman, "International Journal of Systematic Bacteriology" by Shirling and Gottlieb, *18*, 69 and 279 (1968), *19*, 391 (1969) and *22*, 265 (1972), "Bergey's Manual of Determinative Bacteriology", 8th edition (1974), and other published literatures on new species of actinomycetes. In particular, *Streptomyces tenebrarius* is more similar to the strain PA—31088 in respect of the correlation in ability to utilize sugar than S. fradiae. However, some differences are found between the properties of the strain PA—31088 and those of the above strains disclosed in "Antimicrobial Agent and Chemotherapy" 324 (1967). The former has no sclerotium on any medium but the latter has. The former cannot utilize fructose and sucrose but the latter ones can. The former peptonizes milk but does not coagulate it. However, the latter ones both peptonize and coagulate. The former does not grow in gelatin and is mesophilic, showing no growth at a temperature higher than 42°C, but the latter ones are thermophilic, growing better and forming more spores at temperatures of 37 to 50°C than at 28°C. The former is clearly different from the latter ones in microbiological properties, so it is concluded that the strain PA—31088 belongs to a new species of *Streptomyces* and was designated *Streptomyces tokunonensis* sp. nov. The strain PA—31088 has been deposited in the Fermentation Research Institute and American Type Culture Collection as noted above. This invention includes all natural or artificial mutants of the above-described microorganism identified as *Streptomyces tokunonensis*. The artificial production of mutants may be accomplished by a conventional procedure such as X-ray or ultraviolet ray irradiation, nitrogen mustards, 4-nitroquinoline N-oxide, N-methyl-N'-nitro-N-nitrosoguanidine and other mutagens.

The production of the antibiotic PA—31088—IV is carried out by cultivating the PA—31088—IV producing strain or a mutant thereof in an enriched medium under aerobic conditions, whereupon the antibiotic PA—31088—IV is isoalted from the cultured broth. A general method of preparing the antibiotic PA—31088—IV is as follows:

The conditions of fermentation and the composition of the medium follow the usual known procedure for producing antibiotics. The composition of the medium may be varied over a very wide range. The medium essentially contains carbon sources, nitrogen sources and inorganic elements. Vitamins, precursors and other materials to stimulate the fermentation may be added if necessary. Examples of suitable carbon sources are glucose, starch, dextrin, glycerol, molasses or organic acids. They may be used singly or in combination. Examples of nitrogen sources are soybean meal, corn steep liquor, meat extract, yeast extract, cotton seed flour, peptone, wheat germ, ammonium nitrate, which are used singly or in combination. The inorganic elements may be selected from, for example, calcium carbonate, sodium chloride, potassium chloride, magnesium sulfate, cobalt chloride, various phosphates and similar. They are added to the medium if the occasion demands. Liquid media are preferred for production on a large scale.

Fermentation may be carried out under aerobic or submerged aerobic conditions. A submerged aerobic culture is preferable. The pH of the medium may be adjusted to 5.5 to 8.5. A buffering agent such as calcium carbonate may be added to the medium if the pH of the medium varies during the fermentation.

The temperature may be kept at 20 to 40°C, more preferably at 25 to 32°C, during fermentation. The fermentation period depends on the scale. It takes 20 to 80 hours on a large scale. If excessive foaming is encountered during the fermentation, antifoaming agents such as vegetable oil, lard oil, and polypropylene glycol may be added to the medium prior to or in the course of fermentation.

The antibiotic PA—31088—IV can be isolated from the fermentation broth in a per se conventional manner once fermentation has finished. There may be employed any conventional method such as filtration, centrifugation, adsorption and desorption with ion-exchange resins, chromatography with various active adsorbents or extraction with suitable solvents. The procedures may be combined as appropriate.

In order to prevent PA—31088—IV from decomposing, a suitable stabilizing agent may be added during the isolation step, if necessary.

As PA—31088—IV is recovered from the broth, adsorption procedures are preferably used when fermentation is effected on a large scale. For example, the fermentation broth is centrifuged and the supernatant adsorbed on a suitable adsorbent such as active carbon, diatomaceous earth, silica gel, various ion-exchange resins and porous polymers. The elution is effected with a suitable solvent such as water, saline solutions, buffer solutions and similar. The adsorption procedure may be repeated to

obtain a more purified preparation of PA—31088—IV.

Crude PA—31088—IV obtained in this manner may be further purified, if desired, by suitable methods such as reprecipitation, chromatography, lyophilization, and various other methods described above to be used for isolation.

Additionally, PA—31088—IV can be converted into a salt with a base during the isolation procedures and also after isolation. Preferred are the pharmaceutically acceptable salts, for example, the sodium, potassium and calcium salts.

The antibiotic PA—31088—IV is useful as a medicament, a veterinary drug, or as a sterilizer for effective action against gram-positive and gram-negative bacteria including β-lactamase-producing strains. Therefore, PA—31088—IV and its pharmaceutically acceptable salts may be orally or parenterally administered to humans or animals. The antibiotic may be made into tablets, capsules, powder or similar in admixture with diluents, stabilizing agents, preservatives or detergents for oral administration. Further, it may be administered in the form of an injection preparation, as an ointment or as a suppository.

The dosage of PA—31088—IV is generally about 1/10th to several times that of cefalotin, although this depends on the purpose of the treatment. For example, the daily dosage for a human adult is 0.1 to 30 g for a subcutaneous injection.

Since it has a strong β-lactamase inhibiting activity the antibacterial activity of PA—31088—IV is several times greater than that of the 8-lactam hype antibiotics against β-lactamase-producing bacteria. Therefore, PA—31088—IV may be used in combination with the well-known antibiotics of the β-lactam type such as penicillins (e.g. benzylpenicillin, phenoxymethylpenicillin, carbenicillin, ampicillin, amoxicillin) and cephalosporins (e.g. cefaloridine, cefazolin, cefalexin, cefacetrile, cefamandole, cefapirin, cefradine and cefaloglycin).

## Example

(a) Fermentation Process

Medium S (preliminary seed medium): soluble starch 0.5%, glucose 0.5%, polypeptone 0.5%, meat extract 0.5%, yeast extract 0.25%, sodium chloride 0.25%, demineralized water (pH 7.0 before sterilization)

Medium B (fermentation medium): tomato paste 2.4%, dextrin 2.4%, dry yeast 1.2%, cobalt chloride.$6H_2O$ 0.0006%, water (pH 7.0 before sterilization).

To produce a preliminary seed culture, *Streptomyces tokunonensis* PA—31088—IV (FERM—P No. 4843, ATCC No. 31569) is inoculated into a 2-liter Erlenmeyer flask containing 800 ml of Medium S of the above composition and incubated at 28°C for 48 hours under stirring at 180 r.p.m.

The germinated seed broth (800 ml) is inoculated into a 30-liter jar containing 20 liters of Medium B of the above composition and incubated for 65 hours at 28°C with aeration of 20 liters per minute and an internal pressure of 0.5 kg/cm$^2$ under stirring at 150 to 300 r.p.m.

(b) Isolation Process

The fermentation broth is mixed with disodium ethylenediaminetetraacetate (hereinafter abbreviated to EDTA) until the concentration reaches 50 γ/ml. The supernatant fluid (100 liters) separated by means of a Sharples centrifugal separator is cooled to 10°C, adjusted to pH 7.0, passed through a column of 7 liters of Amberlite IRA—68 ($Cl^-$) (by Rohm and Haas Co., USA) at a rate of 600 ml per minute. Amberlite IRA—68 was previously equilibrated to pH 7. The column is eluted with cooled 5% sodium chloride demineralized water. The fractions (12 liters) showing antibacterial activity as checked by the pulp disk diffusion method with Escherichia coli are collected, adjusted to pH 7.0, and passed through a column of 7 liters of Diaion HP—20 (by Mitsubishi Kasei Co.) at a rate of 120 ml per minute. The column is eluted with cooled demineralized water containing 10 γ/ml of EDTA. The active fractions (12 liters) are adjusted to pH 5.0, and 300 g of active carbon are added thereto. The mixture is stirred for 30 minutes and filtered under suction. The carbon cake is washed with water and extracted with 50% cooled acetone containing 10 γ/ml of EDTA. The extrtact is concentrated under reduced pressure to a nearly aqueous solution. The residue is adjusted to pH 7.0 and lyophilized to give approx. 20 g of PA—31088—IV as a crude powder.

(c) Purification Process 1

The crude powder of PA—31088—IV (1 g) prepared as above is dissolved in 5 ml of 10 mM phosphate buffer solution (pH 7.0) containing 10 μg/ml of EDTA. The solution is applied to a column (40 x 350 mm) of Diaion HP—20 and developed with the above buffer solution. The fractions containing PA—31088—IV are collected and sodium chloride is added thereto until the concentration reaches 5% (weight by volume). The solution is adsorbed on a column of Diaion HP—20 and eluted with water containing 10 μg/ml of EDTA and a mixture of water and methanol (1:1). The active fractions are concentrated and lyophilized to give a brown powder (20 mg) containing 10 to 15% of PA—31088—IV. Some 240 mg of the powder can be obtained from 12 g of the starting crude powder.

The resulting powder is dissolved in 2 ml of 10 mM phosphate buffer solution (pH 7.0) containing 10 μg/ml of EDTA. The solution is applied to a column (25 × 500 mm) of Hitachi gel #3019 (by Nissei

Sangyo Co.) and developed with the above buffer solution. Sodium chloride is added to the active fractions until the concentration reaches 5% (weight by volume). The solution is adsorbed on the column (20 x 200 mm) of Diaion HP—20. The column is washed with water containing 1 μg/ml of EDTA and a mixture of water and methanol (1:1). The active eluate fractions are concentrated and lyophilized to give 50 mg of a light yellow powder containing PA—31088—IV in a proportion of 50%.

(d) Purification Process 2

The powder (50 mg) obtained as above is dissolved in 10 mM phosphate buffer solution (pH 7.0) containing 10 μg/ml of EDTA. The solution is subjected to high-performance liquid chromatography on a column (10 x 250 mm) of Nucleocil—5$C_{18}$ (M. Nargel Co., West Germany) with a portion containing 1.5 mg of the powder at a time. The column is eluted with the above buffer solution. PA—31088—IV is detected by tracing the absorption at 240 nm with an ultraviolet absorption detector. The peak fractions containing PA—31088—IV are collected and sodium chloride is added thereto until the concentration reaches 5%. The solution is adsorbed on a column of Diaion HP—20 (20 x 200 mm). The column is eluted with water and a mixture of water and methanol (1:1). The active fractions are concentrated and lyophilized to give 20 mg of substantially pure sodium salt of PA—31088—IV as a pale yellowish powder.

## Claims

1. The antibiotic PA—31088—IV, i.e. 3 - [(2 - acetamidoethenyl) - sulfinyl] - 6 - (2 - hydroxy - 1 - methylethylidene) - 7 - oxo - 1 - azabicyclo[3.2.0]hept - 2 - ene - 2 - carboxylic acid having the structural formula

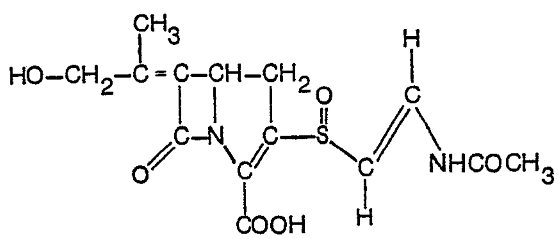

and having the following physicochemical properties:

(a) Elemental analysis
Anal. calcd. for $C_{14}H_{15}N_2O_6S.Na$

| | | | | | |
|---|---|---|---|---|---|
| | C, 46.41; | H, 4.14; | N, 7.73; | S, 8.84; | Na, 6.35 |
| Found: | C, 45.30; | H, 4.82; | N, 7.74; | S, 7.86; | Na, 5.99 |

(b) Mass spectrum of the methyl ester: m/e: 355

(c) Circular dichroism spectrum:
$\lambda_{(nm)}[\theta]$ 390(0), 315(−22800), 278(−103000), 258.5(0), 243(+93300), 190(+14500)

(d) Ultraviolet absorption spectrum (in 10 mM phosphate buffer):
$\lambda^{H_2O}_{max}$ 241 nm ($E^{1\%}_{1 cm}$ 562) (see Fig. 1)

(e) Infrared absorption spectrum:
$\nu_{KBr}$ 3380, 1750, 1695, 1620, 1380, 1270, 1200, 1045, 970 $cm^{-1}$ (see Fig. 2)

(f) Solubility:
Soluble in water, not soluble in acetone, ethyl acetate, chloroform or ether.

(g) Color reaction:
Ninhydrin reaction: negative
Ehrlich's reaction: positive

(h) Acidic substance

(i) $^1$H—NMR spectrum (TMS external reference):
$\delta^{D_2O}_{ppm}$ (Hz) 2.44 (s, 3H), 2.57 (s, 3H), 3.62 (d-like, splitting width 9.4 Hz, 2H), 4.71 (s, 2H), 5.45 (t-like, splitting width 9.4 Hz and 9.4 Hz, 1H), 6.79 (d, 1H, J=14.1), 7.98 (d, 1H, J=14.1)

(j) $^{13}C$—NMR spectrum:

$\delta^{D_2O}_{ppm}$: 15.9q, 23.0q, 32.5t, 60.4t, 64.5t, 111.7d, 134.2s, 134.7d, 135.6s, 143.2s, 150.3s, 166.7s, 173.2s, 174.6s

(k) Thin layer chromatography:
Rf=0.73 (70% ethanol)
Rf=0.30 (80% acetonitrile)
Rf=0.50 (chloroform/ethanol/water (4:7:2))
on cellulose plate (Eastman Co., Ltd.),

and its salts with bases.

2. A process for producing the antibiotic PA—31088—IV as claimed in claim 1, which comprises cultivating a strain of *Streptomyces tokunonensis* or a mutant thereof capable of producing said antibiotic in a nutrient medium under aerobic conditions and then isolating the accumulated antibiotic PA—31088—IV from the cultured broth and, if desired, converting the free acid with a base into its salt.

3. The process claimed in claim 2, wherein the cultivation is effected at a temperature from about 20 to 40°C for about 20 to 80 hours.

4. The antibiotic and its salts with bases according to claim 1 for use in an antibiotic treatment.

5. Pharmaceutical compositions containing the antibiotic or its salt with a base according to claim 1.

6. Streptomyces tokunonensis PA—31088 (FERM—P No. 4843; ATCC No. 31569) and its mutants capable of producing the antibiotic according to Claim 1.

## Revendications

1. L'antibiotique PA—31088—IV, c'est-à-dire l'acide 3 - [(2 - acétamidoéthényl) - sulfinyl] - 6 - (2 - hydroxy - 1 - méthyléthylidène) - 7 - oxo - 1 - azabicyclo[3.2.0] - hept - 2 - ène - 2 - carboxylique répondant à la formule développée:

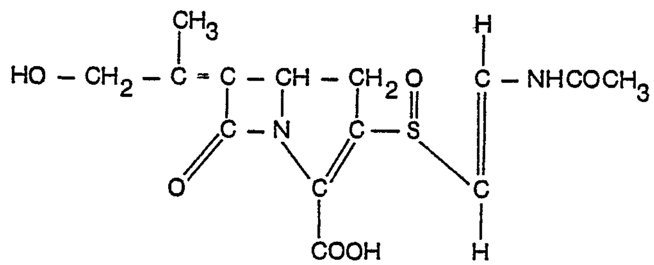

et ayant les propriétés physico-chimiques suivantes:

(a) Analyse élémentaire:
analyse théorique pour: $C_{14}H_{15}N_2O_6S.Na$

| | | | | | |
|---|---|---|---|---|---|
| | C = 46,41; | H = 4,14; | N = 7,73; | S = 8,84; | Na = 6,35 |
| trouvée: | C = 45,30; | H = 4,82; | N = 7,74; | S = 7,86; | Na = 5,99 |

(b) Spectre de masse de l'ester méthylique: m/e: 355

(c) Spectre de dichroïsme circulaire:
$\lambda_{(nm)}[\theta]$ 390(0), 315(−22800), 278(−103000), 258,5(0), 243(+93300), 190(+14500)

(d) Spectre d'absorption ultraviolette (dans du tampon phosphate 10 mM):
$\lambda^{H_2O}_{max}$ 241 nm ($E^{1\%}_{1\ cm}$ 562) (voir fig. 1)

(e) Spectre d'absorption infrarouge:
$\nu_{KBr}$ 3380, 1750, 1695, 1620, 1380, 1270, 1200, 1045, 970 $cm^{-1}$ (voir fig. 2)

(f) Solubilité:
Soluble dans l'eau, insoluble dans l'acétone, l'acétate d'éthyle, le chloroforme et l'éther.

(g) Réactions colorées:
Réaction à la ninhydrine: négative
Réaction d'Ehrlich: positive

(h) Substance acide

(i) Spectre de $^1$H—RMN (TMS comme étalon externe):

$\delta_{ppm}^{D_2O}$ (Hz) 2,44 (s, 3H), 2,57 (s, 3H), 3,62 (de type d, largeur de dédoublement 9,4 Hz, 2H), 4,71 (s, 2H), 5,45 (de type t, largeur de dédoublement 9,4 Hz et 9,4 Hz, 1H), 6,79 (d, 1H, J=14,1), 7,98 (d, 1H, J=14,1)

(j) Spectre de $^{13}$C—RMN:

$\delta_{ppm}^{D_2O}$: 15,9q, 23,0q, 32,5t, 60,4t, 64,5t, 111,7d, 134,2s, 134,7d, 135,6s, 143,2s, 150,3s, 166,7s, 173,2s, 174,6s

(k) Chromatographie en couche mince:
Rf = 0.73 (éthanol à 70%)
Rf = 0.30 (acétonitrile à 80%)
Rf = 0.50 (chloroforme/éthanol/eau (4/7/2) sur plaque de cellulose (Eastman Co., Ltd.) et ses sels formés avec des bases.

2. Un procédé pour produire l'antibiotique PA—31088—IV comme revendiqué dans la revendication 1, qui comprend la culture d'une souche de *Streptomyces tokunonensis* ou d'un de ses mutants capables de produire ledit antibiotique dans un milieu nutritif dans des conditions aérobies puis l'isolement de l'antibiotique PA—31088—IV accumulé du bouillon de culture et, si on le désire, transformation de l'acide libre en son sel avec une base.

3. Le procédé revendiqué dans la revendication 2, dans lequel la culture est effectuée à une température d'environ 20 à 40°C pendant environ 20 à 80 heures.

4. L'antibiotique et ses sels formés avec des bases selon la revendication 1, pour l'utilisation dans une antibiothérapie.

5. Composition pharmaceutique contenant l'antibiotique ou son sel formé avec une base selon la revendication 1.

6. *Streptomyces tokunonensis* PA—31088 (FERM—P n° 4843; ATCC n° 31 569) et ses mutants capables de produire l'antibiotique selon la revendication 1.

**Patentansprüche**

1. Antibiotikum PA—31088—IV, d.h. 3 - [(2 - Acetamidoäthenyl) - sulfinyl] - 6 - (2 - hydroxy - 1 - methyläthyliden) - 7 - oxo - 1 - azabicyclo[3.2.0]hept - 2 - en - 2 - carbonsäure der Formel

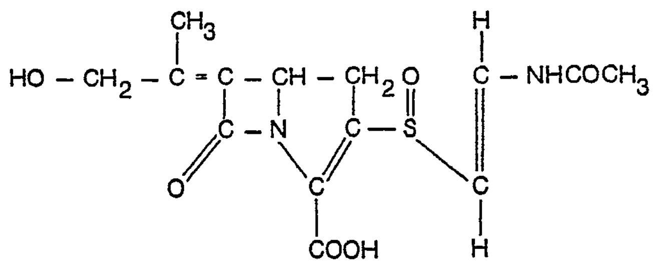

mit folgenden physikalisch-chemischen Eigenschaften:

(a) Elementaranalyse für $C_{14}H_{15}N_2O_6S.Na$:

| | C | H | N | S | Na |
|---|---|---|---|---|---|
| ber.: | 46,41 | 4,14 | 7,73 | 8,84 | 6,35 |
| gef.: | 45,30 | 4,82 | 7,74 | 7,86 | 5,99 |

(b) Massenspektrum des Methylesters: m/e: 355

(c) Circulardichroismus:
$\lambda_{(nm)}[\theta]$ 390(0), 315(−22800), 278(−103000), 258,5(0), 243(+93300), 190(+14500)

(d) UV—Absorptionsspektrum (in 10 mM Phosphatpuffer):
$\lambda_{max}^{H_2O}$ 241 nm ($E_{1\,cm}^{1\%}$ 562) (vgl. Fig. 1)

(e) IR—Absorptionsspektrum:
$\nu_{KBr}$ 3380, 1750, 1695, 1620, 1380, 1270, 1200, 1045, 970 $cm^{-1}$ (vgl. Fig. 2)

(f) Löslichkeit:
löslich in Wasser, unlöslich in Aceton, Äthylacetat, Chloroform oder Äther.

(g) Farbreaktion:
Ninhydrin-Reaktion: negativ
Ehrlich-Reaktion: positiv

(h) saure Substanz

(i) $^{1}H$—NMR Spektrum (TMS äußerer Standard):
$\delta_{ppm}^{D_2O}$ (Hz) 2,44 (s, 3H), 2,57 (s, 3H), 3,62 (d-ähnlich, Spaltbreite 9,4 Hz, 2H), 4,71 (s, 2H), 5,45 (t-ähnlich, Spaltbreite 9,4 Hz und 9,4 Hz, 1H), 6,79 (d, 1H, J=14,1), 7,98 (d, 1H, J=14,1)

(j) $^{13}C$—NMR Spektrum:
$\delta_{ppm}^{D_2O}$: 15,9q, 23,0q, 32,5t, 60,4t, 64,5t, 111,7d, 134,2s, 134,7d, 135,6s, 143,2s, 150,3s, 166,7s, 173,2s, 174,6s

(k) Dünnschichtchromatographie:
$R_f$=0,73 (70% Äthanol)
$R_f$=0,30 (80% Acetonitril)
$R_f$=0,50 (Chloroform/Äthanol/Wasser 4:7:2) an
Celluloseplatte (Eastman Co., Ltd.).

und seine Salze mit Basen.

2. Verfahren zur Herstellung des Antibiotikums PA—31088—IV nach Anspruch 1, dadurch gekennzeichnet, daß man einen zur Bildung des Antibiotikums fähigen Stamm von *Streptomyces tokunonensis* oder dessen Mutante in einem Nährmedium unter aeroben Bedingungen züchtet und das angesammelte Antibiotikum PA—31088—IV aus der Kulturbrühe isoliert und gegebenenfalls die freie Säure mit einer Base in ihr Salz überführt.

3. Verfahren nach Anspruch 2, dadurch gekennzeichnet, daß die Züchtung etwa 20 bis 80 Stunden bei einer Temperatur von etwa 20 bis 40°C durchgeführt wird.

4. Das Antibiotikum und seine Salze mit Basen nach Anspruch 1 zur Verwendung bei einer Behandlung mit Antibiotika.

5. Arzneimittel, enthaltend das Antibiotikum oder dessen Salz mit einer Base gemäß Anspruch 1.

6. Streptomyces tokunonensis PA—31088 (FERM—P Nr. 4843; ATCC Nr. 31 569) und seine Mutanten, die zur Bildung des Antibiotikums gemäß Anspruch 1 fähig sind.

Fig. 1

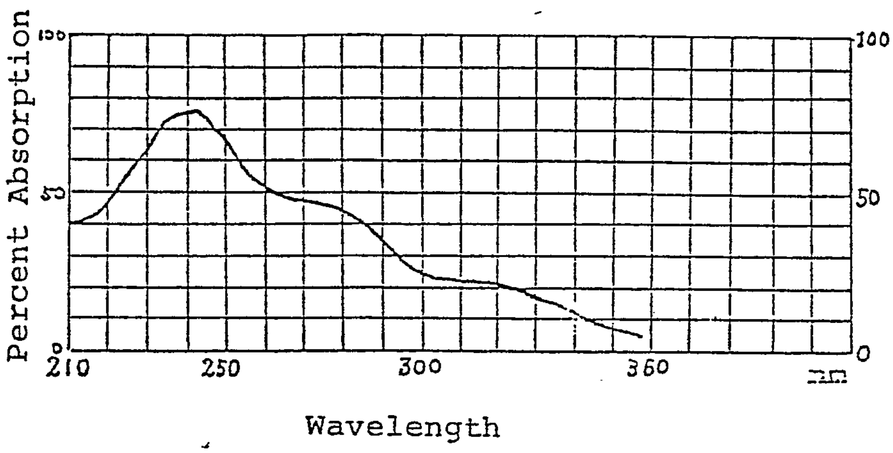
Percent Absorption
100
50
0
100
50
0
210
250
300
360
nm
Wavelength

Fig. 2

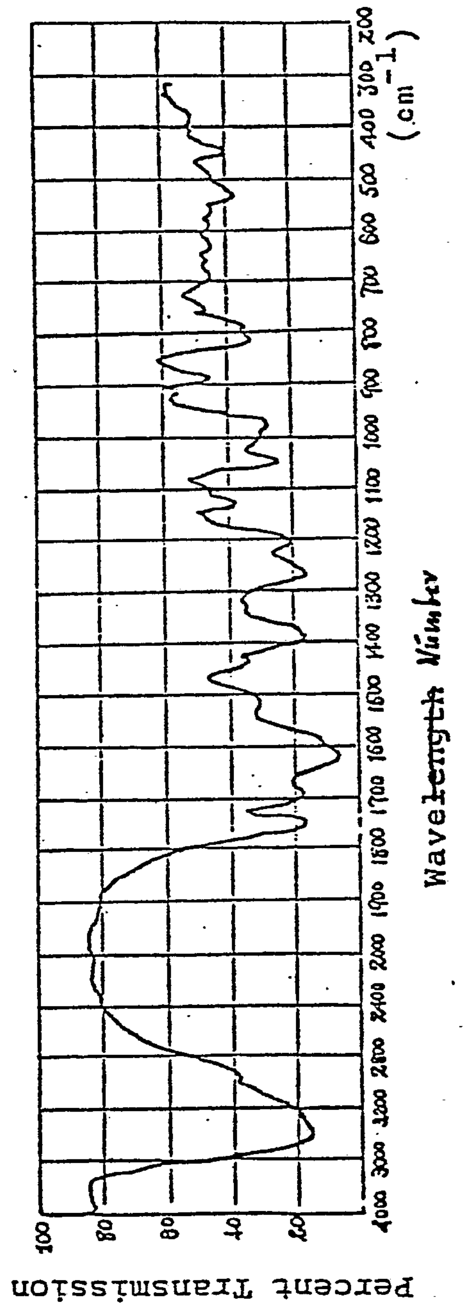

Percent Transmission
100
80
60
40
20
4000 3000 3200 2800 2400 2000 1900 1800 1700 1600 1500 1400 1300 1200 1100 1000 900 800 700 600 500 400 300 200
(cm$^{-1}$)
Wavelength Number